# EUROPEAN PATENT APPLICATION

(11) **EP 4 534 654 A1**
(43) Date of publication of application: **09.04.2025**
(21) Application number: 23383010.8
(22) Date of filing: 02.10.2023
(51) Int. Cl.: C12N 5/0789, A61K 35/28, C12N 15/00

(54) **PRE-STIMULATION METHOD OF CD34+ CELLS TO ACHIEVE CLINICALLY RELEVANT GENE EDITING FREQUENCIES**

(71) Applicant: Consorcio Centro de Investigación Biomédica en Red, 28029 Madrid (ES); Fundacion Instituto De Investigacion Sanitaria Fundacion Jimenez Diaz, 28040 Madrid (ES); Centro de Investigaciones Energéticas, Medioambientales y Tecnológicas O.A., M.P. (CIEMAT), 28040 Madrid (ES); DanausGT Biotechnology Co., Ltd., Jiangsu Province (CN)
(72) Inventor: SÁNCHEZ DOMÍNGUEZ, Rebeca, Madrid (ES); SEGOVIA, José Carlos, Madrid (ES); BONAFONT, José, Madrid (ES); SERRANO, Luis Javier, Madrid (ES); QUINTANA BUSTAMANTE, Óscar, Madrid (ES); OJEDA PÉREZ, Isabel, Madrid (ES)
(74) Representative: Hoffmann Eitle

(57) **Abstract**

The present invention is directed to the different elements that integrate a procedure that shall require the *ex vivo* expansion of HSPCs for, preferably, about 48 hours in the presence of hematopoietic cytokines as well as UM171 and Stemregenin 1 and the introduction of a i) system to generate the double strand breaks of the DNA (DSBs), such as the CRISPR/Cas9 system, upstream of the transcription start site of a target gene, and ii) the introduction of a donor matrix that includes a cDNA flanked by homology arms (Left [LHA] and right [RHA]).

## Description

### TECHNICAL FIELD

The present invention is directed to the different elements that integrate a procedure that shall require the *ex vivo* expansion of HSPCs for, preferably, about 48 hours in the presence of hematopoietic cytokines as well as UM171 and Stemregenin 1 and the introduction of a i) system to generate the double strand breaks of the DNA (DSBs), such as the CRISPR/Cas9 system, upstream of the transcription start site of a target gene, and ii) the introduction of a donor matrix that includes a cDNA flanked by homology arms (Left [LHA] and right [RHA]).

### BACKGROUND ART

Hematopoietic stem cells (HSCs) have the ability to repopulate an entire hematopoietic system (Baum et al., Proceedings of the National Academy of Sciences USA, 89, 2804-2808 (1992)), and several genetic diseases of the blood (Mukherjee and Thrasher, Gene, 525, 174-181 (2013); Cavazzana-Calvo et al., Nature, 467, 318-322 (2010); Naldini, Nature, 526, 351-360 (2015)) and acquired (Jenq & van den Brink, Nature Reviews Cancer, 10, 213-221 (2010)) could potentially be cured by genome editing of HSCs. Recent studies have demonstrated efficient targeted integration in hematopoietic stem and progenitor cells (HSPCs) derived from mobilized peripheral blood, fetal liver, or cord blood by combining ZFN expression with exogenous HR donors delivered via single stranded oligonucleotides (ssODN) (Hoban et al., Blood 125, 2597-2604 (2015)), integrase-defective lentiviral vectors (IDLV) (Genovese et al., Nature 510, 235-240 (2014)), or recombinant adeno-associated viral vectors serotype 6 (rAAV6) (Wang et al., Nature Biotechnology, 33, 1256-1263 (2015)). However, the high editing frequencies in vitro did not result in high frequencies of edited cells following transplantation into immunodeficient mice. The present invention confronts the problem of providing high frequencies of edited cells.

### BRIEF DESCRIPTION OF THE FIGURES

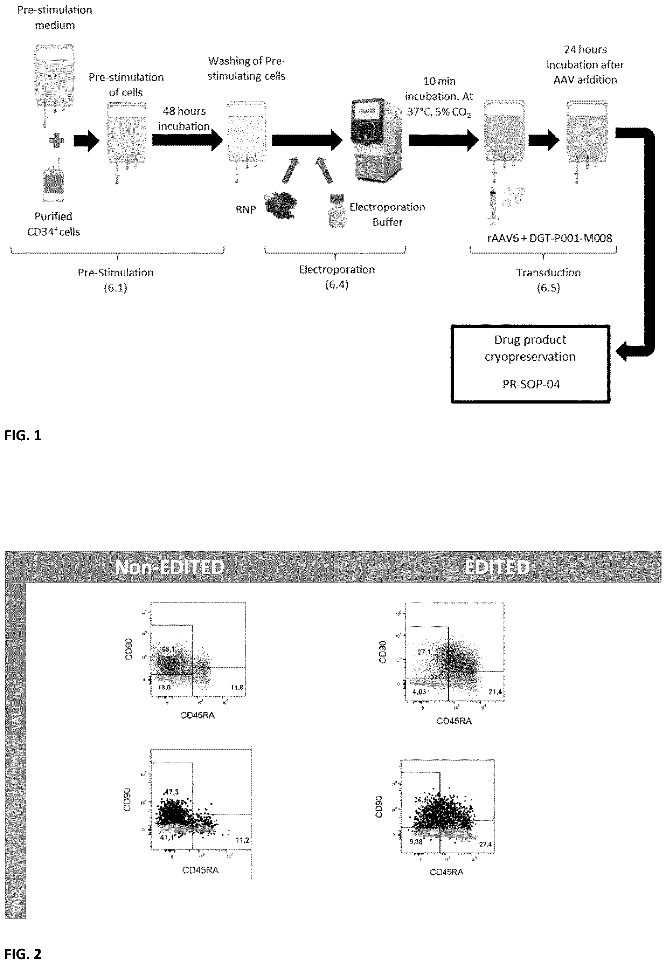

**Fig. 1****. Flow chart of the gene editing manufacturing** process. After purification, cells are pre-stimulated using the defined medium and then electroporated with Cas9/gRNA ribonucleoprotein (RNP), using a commercially available device. Afterwards, cells are transduced with the corresponding AAV vector-carrying genome editing donor sequences and cultured again for additional time. Then, cells are cryopreserved, generating samples for quality control assays and the drug product for infusion in the patient.
**Fig. 2****. Comparison of the hematopoietic stem cell phenotype in non-edited and edited hematopoietic stem and progenitor cells by flow cytometry.** Dot-plots are obtained within the CD34+CD38- gate of the total population. There is an increase of the CD34+CD38-CD90+CD45RA+ population in the edited versus non-edited cells. Two different validation experiments (VAL1 and VAL2) performed at big scale (150 and 300 millions cells edited, respectively).

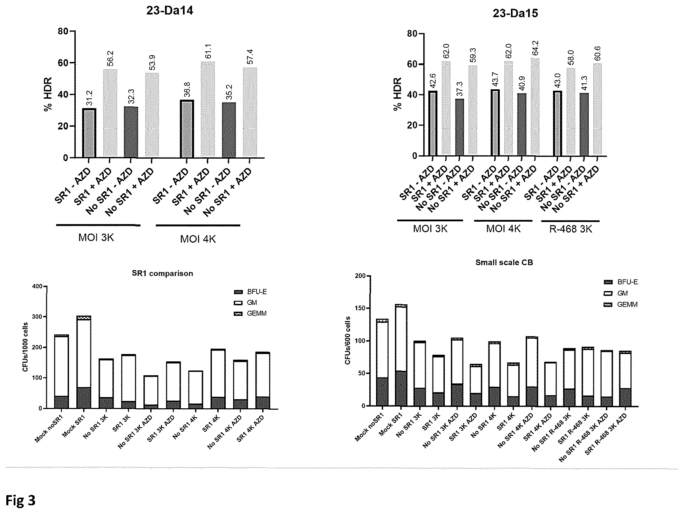

**Fig. 3****. Side by side comparison of different combinations of compounds and different viral doses.** CD34+ cells obtained from mobilized peripheral blood were subjected to the gene editing protocol in the presence or absence of Stemregenin-1 (+/-SR1), AZD-7648 (+/-AZD), and with different viral genomes per cell (MOI) of the AAV vector (3K, 3000; 4K, 4000); R-468 is the identification of a pre-GMP batch. All the other conditions were performed with a cGMP batch. Upper graphs are reflecting gene editing efficiency obtained by specific ddPCR, 5 days after the end of the editing process. Lower graphs represent the colony forming ability of the different samples obtained by semisolid culture. BFU-E, burst forming units-erythroid; GM, granulo-macrophage colony forming units; GEMM, Mixed myeloerythroid colony forming units. Results from two different experiments, named as 23-Da14 and 23-Da15, are showed.

### ABBREVIATIONS AND DEFINITIONS

| | |
|---|---|
| **CRISPR:** | Clustered Regularly Interspaced Short Palindromic Repeats |
| **Cas9:** | CRISPR associated protein 9 |
| **rAAV:** | Recombinant adeno-associated vector |
| **RNP:** | Ribonucleoprotein |
| **HDR:** | Homology-directed repair |
| **sgRNA:** | single guide RNA |
| **MOI:** | Multiplicity of infection |

As used herein, the following terms have the meanings ascribed to them unless specified otherwise.

The terms "a," "an," or "the" as used herein not only include aspects with one member, but also include aspects with more than one member. For instance, the singular forms "a," "an," and "the" include plural referents unless the context clearly dictates otherwise. Thus, for example, reference to "a cell" includes a plurality of such cells and reference to "the agent" includes reference to one or more agents known to those skilled in the art, and so forth.

The term "gene" refers to a combination of polynucleotide elements, that when operatively linked in either a native or recombinant manner, provide some product or function. The term "gene" is to be interpreted broadly, and can encompass mRNA, cDNA, cRNA and genomic DNA forms of a gene.

The term "homology-directed repair" or "HDR" refers to a mechanism in cells to accurately and precisely repair double-strand DNA breaks using a homologous template to guide repair. The most common form of HDR is homologous recombination (HR).

The term "homologous recombination" or "HR" refers to a genetic process in which nucleotide sequences are exchanged between two similar molecules of DNA. Homologous recombination (HR) is used by cells to accurately repair harmful breaks that occur on both strands of DNA, known as double-strand breaks or other breaks that generate overhanging sequences.

The term "single guide RNA" or "sgRNA" refer to a DNA-targeting RNA containing a guide sequence that targets the Cas nuclease to the target genomic DNA and a scaffold sequence that interacts with the Cas nuclease (e.g., tracrRNA), and optionally, a donor repair template.

The term "Cas polypeptide" or "Cas nuclease" refers to a Clustered Regularly Interspaced Short Palindromic Repeats-associated polypeptide or nuclease that cleaves DNA to generate blunt ends at the double-strand break at sites specified by a 20-nucleotide guide sequence contained within a crRNA transcript. A Cas nuclease requires both a crRNA and a tracrRNA for site-specific DNA recognition and cleavage. The crRNA associates, through a region of partial complementarity, with the tracrRNA to guide the Cas nuclease to a region homologous to the crRNA in the target DNA called a "protospacer."

The term "ribonucleoprotein complex" or "RNP complex" refers to a complex comprising an sgRNA and a Cas polypeptide.

The term "homologous donor adeno-associated viral vector" or "donor adeno-associated viral vector" refers to an adeno-associated viral particle that can express a recombinant donor template for CRISPR-based gene editing via homology-directed repair in a host cell, e.g., primary cell.

The term "recombinant donor template" refers to a nucleic acid stand, e.g., DNA strand that is the recipient strand during homologous recombination strand invasion that is initiated by the damaged DNA, in some cases, resulting from a double-stranded break. The donor polynucleotide serves as template material to direct the repair of the damaged DNA region.

The term "primary cell" refers to a cell isolated directly from a multicellular organism. Primary cells typically have undergone very few population doublings and are therefore more representative of the main functional component of the tissue from which they are derived in comparison to continuous (tumor or artificially immortalized) cell lines. In some cases, primary cells are cells that have been isolated and then used immediately. In other cases, primary cells cannot divide indefinitely and thus cannot be cultured for long periods of time in vitro.

The term "gene modified primary cell" or "genome edited primary cell" refers to a primary cell into which a heterologous nucleic acid has been introduced in some cases, into its endogenous genomic DNA.

The term "primary blood cell" refers to a primary cell obtained from blood or a progeny thereof. A primary blood cell can be a stem cell or progenitor cell obtained from blood. For instance, a primary blood cell can be a hematopoietic stem cell or a hematopoietic progenitor cell.

The term "pharmaceutical composition" refers to a composition that is physiologically acceptable and pharmacologically acceptable. In some instances, the composition includes an agent for buffering and preservation in storage, and can include buffers and carriers for appropriate delivery, depending on the route of administration.

The term "pharmaceutical acceptable carrier" refers to a substance that aids the administration of an agent (e.g., Cas nuclease, modified single guide RNA, gene modified primary cell, etc.) to a cell, an organism, or a subject. "Pharmaceutically acceptable carrier" refers to a carrier or excipient that can be included in a composition or formulation and that causes no significant adverse toxicological effect on the patient. Non-limiting examples of pharmaceutically acceptable carrier include water, NaCl, normal saline solutions, lactated Ringer's, normal sucrose, normal glucose, binders, fillers, disintegrants, lubricants, coatings, sweeteners, flavors and colors, and the like. One of skill in the art will recognize that other pharmaceutical carriers are useful in the present invention.

The term "administering or "administration" refers to the process by which agents, compositions, dosage forms and/or combinations disclosed herein are delivered to a subject for treatment or prophylactic purposes. Compositions, dosage forms and/or combinations disclosed herein are administered in accordance with good medical practices taking into account the subject's clinical condition, the site and method of administration, dosage, subject age, sex, body weight, and other factors known to the physician. For example, the terms "administering" or "administration" include providing, giving, dosing and/or prescribing agents, compositions, dosage forms and/or combinations disclosed herein by a clinician or other clinical professional.

The term "treating" refers to an approach for obtaining beneficial or desired results including but not limited to a therapeutic benefit and/or a prophylactic benefit. By therapeutic benefit is meant any therapeutically relevant improvement in or effect on one or more diseases, conditions, or symptoms under treatment. For prophylactic benefit, the compositions may be administered to a subject at risk of developing a particular disease, condition, or symptom, or to a subject reporting one or more of the physiological symptoms of a disease, even though the disease, condition, or symptom may not have yet been manifested.

The terms "culture," "culturing," "grow," "growing," "maintain," "maintaining," "expand," "expanding," etc., when referring to cell culture itself or the process of culturing, can be used interchangeably to mean that a cell (e.g., primary cell) is maintained outside its normal environment under controlled conditions, e.g., under conditions suitable for survival. Cultured cells are allowed to survive, and culturing can result in cell growth, stasis, differentiation or division. The term does not imply that all cells in the culture survive, grow, or divide, as some may naturally die or senesce. Cells are typically cultured in media, which can be changed during the course of the culture.

The terms "subject," "patient," and "individual" are used herein interchangeably to include a human or animal. For example, the animal subject may be a mammal, a primate (e.g., a monkey), a livestock animal (e.g., a horse, a cow, a sheep, a pig, or a goat), a companion animal (e.g., a dog, a cat), a laboratory test animal (e.g., a mouse, a rat, a guinea pig, a bird), an animal of veterinary significance, or an animal of economic significance.

The term "StemSpan AOF" shall be understood as cGMP-cell medium for CD34+ cells cell culture during pre-stimulation step or other process during gene manipulation protocol.

The term "StemSpan SFEM II" shall be understood as cell medium for CD34+ cells cell culture during pre-stimulation step or other process during gene manipulation protocol.

The term "UM171" shall be understood as small molecule for the *ex vivo* expansion of human Hematopoietic Stem Cells. CAS number 1448724-09-1. Chemical or molecular structure:

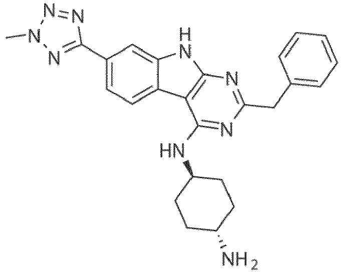

The term "Stemregenin 1" shall be understood as small molecule anti-differentiation/apoptosis used for the in vitro cell culture of human Hematopoietic Stem Cells. CAS No. 1227633-49-9. Chemical or molecular structure:

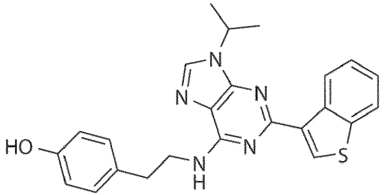

The term "SCF" shall be understood as 'Stem Cell Factor' used for in vitro cell culture of human Hematopoietic Stem Cells related to survival, mobility, and possibly self-renewal of HSCs in culture and in the hematopoiesis stem cell niche.

The term "TPO" shall be understood as 'Thrombopoietin' used for Hematopoietic Stem Cells regulation.

The term "FIt3-Ligand" shall be understood as 'Fms-related tyrosine kinase 3 ligand' is related to ex vivo Hematopoietic Stem Cells expansion.

The term"IL-6" shall be understood as Interleukine-6, is related to Hematopoietic Stem Cells production.

The term "AZD" shall be understood as a DNA-PK inhibitor that could enhance HDR-mediated correction when added during stem cell transduction. CAS No. 2230820-11-6 Chemical or molecular formula:

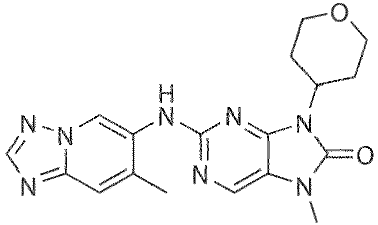

Unless defined otherwise, all technical and scientific terms used herein have the same meanings as commonly understood by one of ordinary skill in the art to which this technology belongs. Although exemplary methods, devices and materials are described herein, any methods and materials similar or equivalent to those expressly described herein can be used in the practice or testing of the present technology. For example, the reagents described herein are merely exemplary and that equivalents of such are known in the art. The practice of the present technology can employ, unless otherwise indicated, conventional techniques of tissue culture, immunology, molecular biology, microbiology, cell biology, and recombinant DNA, which are within the skill of the art. See, e.g., Sambrook and Russell eds. (2001) Molecular Cloning: A Laboratory Manual, 3rd edition; the series Ausubel et al. eds. (2007) Current Protocols in Molecular Biology; the series Methods in Enzymology (Academic Press, Inc., N.Y.); MacPherson et al. (1991) PCR I: A Practical Approach (IRL Press at Oxford University Press); MacPherson et al. (1995) PCR 2: A Practical Approach; Harlow and Lane eds. (1999) Antibodies, A Laboratory Manual; Freshney (2005) Culture of Animal Cells: A Manual of Basic Technique, 5th edition; Miller and Calos eds. (1987) Gene Transfer Vectors for Mammalian Cells (Cold Spring Harbor Laboratory); and Makrides ed. (2003) Gene Transfer and Expression in Mammalian Cells (Cold Spring Harbor Laboratory).

### DESCRIPTION OF EMBODIMENTS

The present invention is directed to the different elements that integrate a procedure that shall require the *ex vivo* expansion of HSPCs for, preferably, about 48 hours in the presence of hematopoietic cytokines as well as UM171 and Stemregenin 1 and the introduction of a i) system to generate the double strand breaks of the DNA (DSBs), such as the CRISPR/Cas9 system, upstream of the transcription start site of a target gene, and ii) the introduction of a donor matrix that includes a cDNA flanked by homology arms (Left [LHA] and right [RHA]). LHA and RHA are identical to the genomic sequences where the exogenous sequences will be inserted. The CRISPR/Cas9 shall be introduced by electroporation to favor the access of DNA nucleases into the cell nucleus, and the donor matrix shall be introduced by means of and adenoassociated viral vector, preferably of serotype 6. To assemble the ribonucleoproteins (RNP), Cas9 protein is combined with the sgRNA. For the nuclefection of RNP into the cell, an electroporator device is used. Cells are pre-stimulated then resuspended in electroporation solution. RNP complex is added into the cellular suspension and the cells are electroporated. After the electric pulse, HSPCs are incubated for 10 minutes at 37°C. Then, a pre-warmed medium is added, and cells are transferred to a culture system. Nucleofected cells are immediately transduced with the corresponding AAV at different concentrations.

The cells, primary cells such as Hematopoietic Stem Cells (HSC) are therefore obtained from a patient, manipulated *in vitro,* and once the product has been characterized and demonstrated that meets the acceptance criteria for drug product release, the cells infused in the patient who has been previously conditioned with chemotherapy to allow the engraftment of the infused corrected cells.

The present invention is based on the surprising discovery that the method (from hereinafter "stimulating method of the invention") for stimulating the *ex vivo* expansion of primary cells such as Hematopoietic stem/progenitor cells (HSPCs) of the invention, has a considerable impact on HDR efficacy directly when used as a pre-stimulation step in a method for inducing a stable gene modification of a target nucleic acid via homologous recombination in a cell population comprising primary cells. In fact, among other advantageous effects, the established pre-stimulation time and the type of media used facilitates HSCs to enter in Cell Cycle, which increases HDR frequency. The present invention thus confronts the problem of providing high frequencies of edited cells.

Therefore, in some aspects, provided herein is a method (from hereinafter "stimulating method of the invention") for stimulating the *ex vivo* expansion of primary cells such as Hematopoietic stem/progenitor cells (HSPCs), the method comprising culturing in an appropriate basal medium such as StemSpan AOF or StemSpan SFEM II, or any other cell medium for CD34+ cells cell culture, supplemented with UM171 and Stemregenin 1, and cytokines and optionally further compounds, the primary cells. Preferably, in a specific embodiment, the cytokines and further compounds are selected from any one of the list consisting of: SCF, TPO, FIt3-Ligand, IL3 and IL-6. More preferably, UM171 is added to the media at a final concentration of ±30%, ±20%, ±10%, ±5% or ±1% of 35 nM and Stemregenin 1 is also added to the media at a final concentration of ±30%, ±20%, ±10%, ±5% or ±1% of 0.75 mM.

It is herein noted that although different stimulation media can be used for the *ex vivo* expansion of primary cells such as Hematopoietic stem/progenitor cells (HSPCs), the addition of UM171 and Stemregenin 1 to an appropriate basal medium such as StemSpan AOF or StemSpan SFEM II, or any cell medium for CD34+ cells cell culture, supplemented with cytokines and optionally further compounds, significantly stimulates the *ex vivo* expansion of primary cells such as HSPCs.

Preferably, in another specific embodiment, the stimulating method of the invention comprises culturing in an appropriate basal medium, such as StemSpan AOF or StemSpan SFEM II, supplemented with UM171 and Stemregenin 1 and the following cytokines and further compounds: SCF, TPO, Flt3-Ligand, IL3 and IL-6, the primary cells. Preferably, wherein the cytokines and further compounds are present in the media at a final concentration of ±30%, ±20%, ±10%, ±5% or ±1% of any of the values of the range of from 100 to 300ug/ml SCF, ±30%, ±20%, ±10%, ±5% or ±1% of any of the values of the range of from 100 to 300ug/ml FLT3, ±30%, ±20%, ±10%, ±5% or ±1% of any of the values of the range of from 100 to 300ug/ml TPO, ±30%, ±20%, ±10%, ±5% or ±1% of any of the values of the range of from 100 to 300ug/ml IL6, ±30%, ±20%, ±10%, ±5% or ±1% of any of the values of the range of from 100 to 300ug/ml IL3. More preferably, wherein the cytokines and further compounds are present in the media at a final concentration of 30%, ±20%, ±10%, ±5% or ±1% of any of the values of the range of from 100 to 300ug/ml SCF, ±30%, ±20%, ±10%, ±5% or ±1% of any of the values of the range of from 100 to 300ug/ml FLT3, ±30%, ±20%, ±10%, ±5% or ±1% of any of the values of the range of from 100 to 300ug/ml TPO, ±30%, ±20%, ±10%, ±5% or ±1% of any of the values of the range of from 100 to 300ug/ml IL6, and ±30%, ±20%, ±10%, ±5% or ±1% of any of the values of the range of from 100 to 300ug/ml IL3 and the media is StemSpan AOF or StemSpan SFEM II, more preferably StemSpan AOF or any cell medium for CD34+ cells cell culture. Still more preferably, wherein the cytokines and further compounds are present in the media at a final concentration of ±30%, ±20%, ±10%, ±5% or ±1% of 100ug/ml SCF, ±30%, ±20%, ±10%, ±5% or ±1% of 100ug/ml FLT3, ±30%, ±20%, ±10%, ±5% or ±1% of 100ug/ml TPO, and ±30%, ±20%, ±10%, ±5% or ±1% of 100ug/ml IL6, the media is StemSpan AOF or StemSpan SFEM II, more preferably StemSpan AOF, and UM171 is added to the media at a final concentration of ±30%, ±20%, ±10%, ±5% or ±1% of 35 nM and Stemregenin 1 is also added to the media at a final concentration of ±30%, ±20%, ±10%, ±5% or ±1% of 0.75 nM. Still more preferably, wherein the cytokines are present in the media at a final concentration of 100ug/ml SCF, 100ug/ml FLT3, 100ug/ml TPO, and 100ug/ml IL6, the media is StemSpan AOF or StemSpan SFEM II, more preferably StemSpan AOF, and UM171 is added to the media at a final concentration of 35 nM and Stemregenin 1 is also added to the media at a final concentration of 0.75 nM.

It is noted that preferably in the stimulating method of the invention the primary cells, preferably isolated CD34+ HSPCs, are not cryopreserved prior to the pre-stimulation step as hematopoietic progenitor viability is affected by cryopreservation.

It is further noted that the established stimulation time facilitates primary cells such as HSCs to enter in Cell Cycle, which increases HDR frequency. In particular, the primary cells, preferably isolated CD34+ HSPCs, are culture in accordance with the stimulating method of the invention for 24 hours or more, preferably for 48 ± 6 hours.

In some embodiments, the primary cell is selected from the group consisting of a primary blood cell, a primary mesenchymal cell, and a combination thereof. In some embodiments, the primary blood cell is selected from the group consisting of an immune cell, a red blood cell, a progenitor or stem cell thereof, and a combination thereof. In some instances, the immune cell is selected from the group consisting of a T cell, a B cell, a dendritic cell, a natural killer cell, a macrophage, a neutrophil, an eosinophil, a basophil, a mast cell, a precursor thereof, and a combination thereof. The progenitor or stem cell can be selected from the group consisting of a hematopoietic progenitor cell, a hematopoietic stem cell, and a combination thereof. In some cases, the red blood cell is a blood stem cell. In some instances, the primary mesenchymal cell is selected from the group consisting of a mesenchymal stem cell, a mesenchymal progenitor cell, a mesenchymal precursor cell, a differentiated mesenchymal cell, and a combination thereof. The differentiated mesenchymal cell can be selected from the group consisting of a bone cell, a cartilage cell, a muscle cell, an adipose cell, a stromal cell, a fibroblast, a dermal cell, and a combination thereof. Preferably, the primary cell is a CD34+ HSPC (Hematopoietic stem and progenitor cell).

Another aspect of the invention is directed to a method for inducing a stable gene modification of a target nucleic acid via homologous recombination in a population of primary cells, the method comprising:
a) Pre-stimulating the population of primary cells such as CD34+ HSPCs in accordance with the stimulating method of the invention for 24 hours or more, preferably for 48:t6 hours,
b) Preferably resuspend the population of primary cells in an electroporation solution;
c) introducing into the pre-stimulated population of primary cells:
   a. a modified single guide RNA (sgRNA) comprising a first nucleotide sequence that is complementary to the target nucleic acid and a second nucleotide sequence that interacts with a CRISPR-associated protein (Cas) polypeptide;
   b. a Cas polypeptide, an mRNA encoding a Cas polypeptide, and/or a recombinant expression vector comprising a nucleotide sequence encoding a Cas polypeptide, wherein the modified sgRNA guides the Cas polypeptide to the target nucleic acid; and
   c. a homologous donor adeno-associated viral (AAV) vector comprising a recombinant donor template comprising two nucleotide sequences comprising two non-overlapping, homologous portions of the target nucleic acid, wherein the nucleotide sequences are located at the 5' and 3' ends of a nucleotide sequence corresponding to the target nucleic acid to undergo homologous recombination.

In particular, after step a) the population of primary cells is resuspended in electroporation solution. RNP complex is added into the cellular suspension and the cells are electroporated. After the electric pulse, the cell population comprising primary cells such as HSPCs are incubated for about 5 to 30 minutes at about 37°C. Then, a pre-warmed medium is added, and cells are transferred to a cell culture system. Nucleofected cells are immediately transduced with the corresponding AAV (preferably within 30 minutes after electroporation) at different concentrations.

In some embodiments, the primary cell is isolated from a mammal prior to introducing the modified sgRNA, the Cas polypeptide, and the homologous donor AAV vector into the primary cell. For instance, the primary cell can be harvested from a human subject. In some instances, the primary cell or a progeny thereof is returned to the mammal after introducing the modified sgRNA, the Cas polypeptide, and the homologous donor AAV vector into the primary cell. In other words, the genetically modified primary cell undergoes autologous transplantation. In other instances, the genetically modified primary cell undergoes allogeneic transplantation. For example, a primary cell that has not undergone stable gene modification is isolated from a donor subject, and then the genetically modified primary cell is transplanted into a recipient subject who is different than the donor subject.

The primary cell can comprise a population of primary cells. In some cases, the population of primary cells comprises a heterogeneous population of primary cells. In other cases, the population of primary cells comprises a homogeneous population of primary cells. Preferably, the population of primary cells comprises CD34+ HSPCs.

In some embodiments, the homologous donor AAV vector is selected from a wild-type AAV serotype 1 (AAV1), wild-type AAV serotype 2 (AAV2), wild-type AAV serotype 3 (AAV3), wild-type AAV serotype 4 (AAV4), wild-type AAV serotype 5 (AAV5), wild-type AAV serotype 6 (AAV6), wild-type AAV serotype 7 (AAV7), wild-type AAV serotype 8 (AAV8), wild-type AAV serotype 9 (AAV9), wild-type AAV serotype 10 (AAV10), wild-type AAV serotype 11 (AAV11), wild-type AAV serotype 12 (AAV12), a variant thereof, and any shuffled chimera thereof. In some instances, the homologous donor AAV vector has at least about 90% sequence identity to any one selected from the group consisting of an AAV1, AAV2, AAV3, AAV4, AAV3, AAV5, AAV6, AAV7, AAV8, AAV9, AAV10, AAV11 and AAV12. In other instances, the homologous donor AAV vector is a wild-type AAV6 or an AAV6 variant having at least 95% sequence identity to wild-type AAV6, e.g., 95%, 96%, 97%, 98%, 99%, or 100% sequence identity to wild-type AAV6.

In some embodiments, the stable gene modification of the target nucleic acid is induced in greater than about 70% of the population of primary cells, e.g., about 71%, about 72%, about 73%, about 74%, about 75%, about 76%, about 77%, about 78%, about 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%, or about 100% of the population of primary cells. In other embodiments, the stable gene modification of the target nucleic acid is induced in greater than about 80% of the population of primary cells, e.g., about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%, or about 100% of the population of primary cells. In yet other embodiments, the stable gene modification of the target nucleic acid is induced in greater than about 90% of the population of primary cells, e.g., about 90%, about 91%, about 92%, about 93%, about 94%, about 95%, about 96%, about 97%, about 98%, about 99%, or about 100% of the population of primary cells.

In other embodiments, the stable gene modification of the target nucleic acid comprises the replacement of a genetic mutation in the target nucleic acid (e.g., to correct a point mutation or a single nucleotide polymorphism (SNP) in the target nucleic acid that is associated with a disease) or the insertion of an open reading frame (ORF) comprising a normal copy of the target nucleic acid (e.g., to knock in a wild-type cDNA of the target nucleic acid that is associated with a disease).

In some embodiments, the Cas polypeptide is a Cas9 polypeptide, a variant thereof, or a fragment thereof. In certain instances, the Cas polypeptide variant comprises a high-fidelity or enhanced specificity Cas9 polypeptide variant. In certain embodiments, the modified sgRNA and the Cas polypeptide are introduced into the primary cell concomitantly. In other embodiments, the modified sgRNA and the Cas polypeptide are introduced into the primary cell sequentially. In some cases, the modified sgRNA is introduced first, and the Cas polypeptide thereafter. In other cases, the Cas polypeptide is introduced first, and the modified sgRNA thereafter.

In some embodiments, the sgRNA and the Cas polypeptide can be incubated together to form a ribonucleoprotein (RNP) complex prior to introducing into the primary cell. For instance, the modified sgRNA and the Cas polypeptide can be mixed together in a vessel to form an RNP complex, and then the RNP complex is introduced into the primary cell. In other embodiments, the Cas polypeptide described herein can be an mRNA encoding the Cas polypeptide, which Cas mRNA is introduced into the primary cell together with the modified sgRNA as an "All RNA" CRISPR system. In certain instances, the modified sgRNA and the Cas mRNA are introduced into the primary cell concomitantly. In other instances, the modified sgRNA and the Cas mRNA are introduced into the primary cell sequentially. In some cases, the modified sgRNA is introduced first, and the Cas mRNA thereafter. In other cases, the Cas mRNA is introduced first, and the modified sgRNA thereafter.

In some embodiments, the RNP complex and the homologous donor AAV vector are concomitantly introduced into the primary cell. In other embodiments, the RNP complex and the homologous donor AAV vector are sequentially introduced into the primary cell. In some instances, the RNP complex is introduced into the primary cell before the homologous donor AAV vector. In other instances, the homologous donor AAV vector is introduced into the primary cell before the RNP complex. For example, the RNP complex can be introduced into the primary cell about 1, 2, 3, 4, 5, 6, 7, 8, 9, 10, 11, 12, 13, 14, 15, 16, 17, 18, 19, 20, 25, 30, 35, 40, 45, 50, 55, 60, 90, 120, 150, 180, 210, or 240 minutes or more before the homologous donor AAV vector, or vice versa. In particular embodiments, the RNP complex is introduced into the primary cell about 15 minutes (e.g., from about 10 to about 20 minutes) before the homologous donor AAV vector.

In some embodiments, the "All RNA" CRISPR system and the homologous donor AAV vector are concomitantly introduced into the primary cell. In other embodiments, the "All RNA" CRISPR system and the homologous donor AAV vector are sequentially introduced into the primary cell. In some instances, the "All RNA" CRISPR system is introduced into the primary cell before the homologous donor AAV vector. In other instances, the homologous donor AAV vector is introduced into the primary cell before the "All RNA" CRISPR system. For example, the "All RNA" CRISPR system can be introduced into the primary cell about 1, 2, 3, 4, 5, 6, 7, 8, 9, 10, 11, 12, 13, 14, 15, 16, 17, 18, 19, 20, 25, 30, 35, 40, 45, 50, 55, 60, 90, 120, 150, 180, 210, or 240 minutes or more before the homologous donor AAV vector, or vice versa. In particular embodiments, the "All RNA" CRISPR system is introduced into the primary cell about 15 minutes (e.g., from about 10 to about 20 minutes) before the homologous donor AAV vector.

In some embodiments, the recombinant donor template also contains a nucleotide sequence encoding a marker selected from the group consisting of a selectable marker, a detectable marker, a cell surface marker, or a combination thereof.

In some embodiments, any of the methods described herein can also include purifying the primary cell having the stable gene modification of the target nucleic acid using the marker. In some cases, the composition isolated by the purifying step includes at least about 80% primary cells having the stable gene modification of the target nucleic acid, e.g., about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%, or more primary cells having the stable gene modification of the target nucleic acid.

In some embodiments, the step of introducing the sgRNA and the Cas polypeptide into the primary cell comprises electroporating the modified sgRNA and the Cas polypeptide into the primary cell. In some embodiments, the step of introducing the homologous donor AAV vector into the primary cell comprises transducing the primary cell. In some embodiments, prior to simultaneously to contacting the cells with the recombinant donor template, such cells are contacted with compound AZD.

In other aspects, provided herein is a genetically modified primary cell produced by any of the methods described herein. In some embodiments, the genetically modified primary cell is selected from the group consisting of a genetically modified primary blood cell, a genetically modified primary mesenchymal cell, and a combination thereof. In some embodiments, the genetically modified primary blood cell is selected from the group consisting of a genetically modified immune cell, a genetically modified red blood cell, a genetically modified progenitor or stem cell thereof, and a combination thereof. In some instances, the genetically modified immune cell is selected from the group consisting of a genetically modified T cell, a genetically modified B cell, a genetically modified dendritic cell, a genetically modified natural killer cell, a genetically modified macrophage, a genetically modified neutrophil, a genetically modified eosinophil, a genetically modified basophil, a genetically modified mast cell, a precursor thereof, and a combination thereof. The genetically modified progenitor or stem cell can be selected from the group consisting of a genetically modified hematopoietic progenitor cell, a genetically modified hematopoietic stem cell, and a combination thereof. In some cases, the genetically modified red blood cell is a genetically modified blood stem cell. In some instances, the genetically modified primary mesenchymal cell is selected from the group consisting of a genetically modified mesenchymal stem cell, a genetically modified mesenchymal progenitor cell, a genetically modified mesenchymal precursor cell, a genetically modified differentiated mesenchymal cell, and a combination thereof. The genetically modified differentiated mesenchymal cell can be selected from the group consisting of a genetically modified bone cell, a genetically modified cartilage cell, a genetically modified muscle cell, a genetically modified adipose cell, a genetically modified stromal cell, a genetically modified fibroblast, a genetically modified dermal cell, and a combination thereof.

In another aspect, the population of genetically modified cells produced by stimulating the *ex vivo* expansion of primary cells such as Hematopoietic stem/progenitor cells (HSPCs) of the invention, when used as a pre-stimulation step in a method for inducing a stable gene modification of a target nucleic acid via homologous recombination in a cell population comprising primary cells, is characterized by comprising a double positive CD45RA+CD90+ population that appears in the edited cells and is absent in unmanipulated cells.

In yet other aspects, provided herein is a pharmaceutical composition comprising any of the genetically modified primary cells described herein, and a pharmaceutically acceptable carrier. In other embodiments, the pharmaceutical composition comprises one type of genetically modified primary cell. In other embodiments, the pharmaceutical composition comprises two or more different types of genetically modified primary cells, e.g., 2, 3, 4, 5, 6, 7, 8, 9, 10 or more different types of genetically modified primary cells.

### DETAILED DESCRIPTION OF THE INVENTION

The present invention discloses an improved method for genetically modifying CD34+ cells by electroporation and transduction with a sgRNA/Cas9/AAV vector system. This improved method provides for a detailed protocol for pre-stimulation, electroporation and transduction of CD34+ cells with AAV to achieve clinically relevant gene editing frequencies. Precise gene editing is in clinical use for several diseases, and more applications are under development. The programmable nuclease Cas9, directed by a single-guide RNA (sgRNA), can introduce double-strand breaks (DSBs) in target sites of genomic DNA, which constitutes the initial step of gene editing using this technology. In the present invention, the repair of the DSBs by homology-directed repair (HDR) is a crucial step to providing a successful outcome of the gene editing strategy.

In particular, in this invention a combination of CRISPR-Cas9 system and donor recombinant adeno-associated vector (rAAV) delivery are used to build an efficient, safe and clinically applicable system to knock-in therapeutic sequences at the translation start site of some genes in human hematopoietic progenitors. Currently, there are some methods to introduce the ribonucleoprotein (RNP) form of the CRISPR-Cas9 system into the cell, being electroporation the choice of preference. Electroporation induces small pores in the cellular membrane allowing the internalization of some small molecules such as RNP. In the same way, rAAV6 is a good tool for homology-directed repair (HDR) donor template delivery.

The present method can be carried by using the process generally illustrated in figure 1. It is noted that the present procedure is carried out by first isolating the CD34+ cells, preferably from mobilized peripheral blood, more preferably by using Sepax C-Pro (CD34 labelling) and CliniMACS Plus (CD34 enrichment process) (PR-SOP-03, CD34 selection in CliniMACS plus). The method is further detailed below.

### CD34 Enrichment and Pre-stimulation

The isolated CD34+ HSPCs, preferably from mobilized peripheral blood, are cultured in an appropriate basal medium such as StemSpan AOF or StemSpan SFEM II (Stemcell Technologies, Vancouver, Canada) supplemented with SCF (100 ng/ml), TPO (100 ng/ml), FIt3-Ligand (100 ng/ml), IL-6 (100 ng/ml), and StemRegeninl (0.75 mM). In addition, UM171 (Stemcell Technologies) is added to the media at a final concentration of 35 nM. Cells were cultured at 37° C., 5% CO2, and 5% 02 for 48±6 hours.

It is noted that preferably the isolated CD34+ HSPCs are not freeze prior to the pre-stimulation step as hematopoietic progenitor viability is affected by freezing. In addition, although different pre-stimulation media can be used it is noted that the addition of UM171 and Stemregenin 1 significantly stimulates the *ex vivo* expansion of HSPCs. In fact, the introduction of protocols using UM171 and SR-1 to culture HSPCs has achieved the successful in vitro expansion of repopulating cells. Moreover, the addition to an appropriate basal medium such as StemSpan AOF or StemSpan SFEM II (Stemcell Technologies, Vancouver, Canada) of SCF (100 ng/ml), TPO (100 ng/ml), FIt3-Ligand (100 ng/ml), IL-6 (100 ng/ml), and StemRegeninl (0.75 mM), and further supplemented with UM171 at a final concentration of 35 nM is the most compelling strategy tested here, as it represents the best compromise between high editing efficiency and preservation of BM repopulating cells.

It is further noted that the established pre-stimulation time facilitates HSCs to enter in Cell Cycle, which increases HDR frequency.

### Electroporation and Transduction

Cas9 RNP was made by incubating protein with sgRNA at a molar ratio of 1:3 at 25° C. for at least 10 minutes immediately prior to electroporation.

Then, CD34+ HSPCs were electroporated after the pre-stimulation step. CD34+ HSPCs were electroporated using the Maxcyte Electroporation flow system with the following conditions: 200-100×10⁶ cells/ml, 300-350 ug/ml Cas9 protein complexed with sgRNA at 1:3 molar ratio. Following electroporation, cells were incubated for 10 min at 37° C. after which they were added AAV donor vectors (generally at an MOI of 4,000 unless otherwise specified), incubated at 37° C. for 24±6 hours. It is noted that surprising, as indicated in figure 3, the further exposition of the cells to compound AZD prior to or during the transduction significantly increased the editing efficiency while preserving the BM in the cells.

### Measuring Targeted Integration

Rates of targeted integration was measured by ddPCR at least 3 days in liquid culture after electroporation. Targeted integration of an coRPK expression cassette was measured by ddPCR using in-out specific primers to detect proper integration.

According to the results obtained, the CD34 Enrichment and Pre-stimulation medium used in the present invention significantly stimulates the *ex vivo* expansion of HSPCs thus having a considerable impact on HDR efficacy directly. In fact, the population of gene edited cells obtained after the Electroporation and Transduction step is characterized by having a double positive CD34+CD38-CD45RA+CD90+ population that appears in the edited cells and is absent in the unmanipulated cells. Please refer to figure 2.

## Claims

1. An *in vitro* method for stimulating the *ex vivo* expansion of primary cells, the method comprising culturing in an appropriate basal medium supplemented with UM171 and Stemregenin 1, the primary cells at preferably 37°C, 5% CO2, and 5% 02 for 48±6 hours.

2. The *in vitro* method according to claim 1, wherein the appropriate basal medium is further supplemented with a compound selected from any one of the list consisting of: SCF, TPO, Flt3-Ligand, IL3 and IL-6.

3. The *in vitro* method according to any one of claims 1 or 2, wherein UM171 is added to the media at a final concentration of ±30%, ±20%, ±10%, ±5% or ±1% of 35 nM and Stemregenin 1 is added to the media at a final concentration of ±30%, ±20%, ±10%, ±5% or ±1% of 0.75 nM.

4. The *in vitro* method according to claim 3, wherein the appropriate basal medium is further supplemented with at least SCF, TPO, FIt3-Ligand, and IL-6.

5. The *in vitro* method according to claim 4, wherein the appropriate basal medium is further supplemented with a final concentration of ±30%, ±20%, ±10%, ±5% or ±1% of 100ug/ml SCF, ±30%, ±20%, ±10%, ±5% or ±1% of 100ug/ml FLT3, ±30%, ±20%, ±10%, ±5% or ±1% of 100ug/ml TPO, and ±30%, ±20%, ±10%, ±5% or ±1% of 100ug/ml IL6.

6. The in vitro method according to claim 5, wherein the media is StemSpan AOF or StemSpan SFEM II, more preferably StemSpan AOF.

7. The in vitro method according to claim 1, wherein the appropriate basal medium is supplemented with a final concentration of 100ug/ml SCF, 100ug/ml FLT3, 100ug/ml TPO, and 100ug/ml IL6, the media is StemSpan AOF, and UM171 is added to the media at a final concentration of 35 nM and Stemregenin 1 is added to the media at a final concentration of 0.75 nM.

8. The in vitro method according to any one of claims 1 to 7, wherein the primary cells are not freeze prior to stimulating the *ex vivo* expansion of primary cells.

9. A method for inducing a stable gene modification of a target nucleic acid via homologous recombination in a population of primary cells, the method comprising:
a. Pre-stimulating the population of primary cells in accordance with the method of any one of claims 1 to 8,
b. introducing into the pre-stimulated population of primary cells:
i. a modified single guide RNA (sgRNA) comprising a first nucleotide sequence that is complementary to the target nucleic acid and a second nucleotide sequence that interacts with a CRISPR-associated protein (Cas) polypeptide;
ii. a Cas polypeptide, an mRNA encoding a Cas polypeptide, and/or a recombinant expression vector comprising a nucleotide sequence encoding a Cas polypeptide, wherein the modified sgRNA guides the Cas polypeptide to the target nucleic acid; and
iii. a homologous donor adeno-associated viral (AAV) vector comprising a recombinant donor template comprising two nucleotide sequences comprising two non-overlapping, homologous portions of the target nucleic acid, wherein the nucleotide sequences are located at the 5' and 3' ends of a nucleotide sequence corresponding to the target nucleic acid to undergo homologous recombination.

10. The method of any one of claims 1 to 9, wherein the primary cell is a cell population comprising CD34+ HSPCs, preferably from mobilized peripheral blood.

11. The method of any one of claims 1 to 10, wherein the homologous donor AAV vector is selected from a wild-type AAV serotype 1 (AAV1), wild-type AAV serotype 2 (AAV2), wild-type AAV serotype 3 (AAV3), wild-type AAV serotype 4 (AAV4), wild-type AAV serotype 5 (AAV5), wild-type AAV serotype 6 (AAV6), wild-type AAV serotype 7 (AAV7), wild-type AAV serotype 8 (AAV8), wild-type AAV serotype 9 (AAV9), wild-type AAV serotype 10 (AAV10), wild-type AAV serotype 11 (AAV11), wild-type AAV serotype 12 (AAV12), a variant thereof, and any shuffled chimera thereof.

12. The method of any one of claims 9 to 11, wherein the modified sgRNA and the Cas polypeptide are incubated together to form a ribonucleoprotein (RNP) complex prior to introducing into the primary cell.

13. The method of any one of claims 9 to 12, wherein the cells are prior to or simultaneously to the transduction with AAV exposed or contacted with compound AZD.

14. A genetically modified primary cell produced by the method of any one of claims 1 to 13.

15. A composition, preferably a pharmaceutical composition, comprising the genetically modified primary cell of claim 14, for use in a method for preventing or treating a disease in a subject in need thereof, the method comprising administering to the subject the genetically modified primary cell of claim 13 to prevent the disease or ameliorate one or more symptoms of the disease.

16. The composition for use according to claim 15, wherein the disease is selected from the group consisting of a pyruvate kinase deficiency, hemoglobinopathy, a viral infection, X-linked severe combined immune deficiency, Fanconi anemia, hemophilia, neoplasia, cancer, amyotrophic lateral sclerosis, Alzheimer's disease, Parkinson's disease, cystic fibrosis, blood diseases and disorders, inflammation, immune system diseases or disorders, metabolic diseases, liver diseases and disorders, kidney diseases and disorders, muscular diseases and disorders, bone or cartilage diseases and disorders, neurological and neuronal diseases and disorders, cardiovascular diseases and disorders, pulmonary diseases and disorders, and lysosomal storage disorders.
